# EUROPÄISCHE PATENTSCHRIFT

(11) **EP 2 020 223 B1**
(45) Veröffentlichungstag und Bekanntmachung des Hinweises auf die Patenterteilung: **12.09.2012**
(21) Anmeldenummer: 08161287.1
(22) Anmeldetag: 28.07.2008
(51) Int. Cl.: A61K 8/44, A61Q 5/12, A61K 8/19, A61K 8/04, A61Q 5/02, A61K 8/46

(54) **Haarbehandlungsmittel mit einem Gehalt an Diamanten**
Hair treatment agent containing diamonds
Produit de soin capillaire ayant une teneur en diamants

(30) Priorität: 01.08.2007 DE 102007037122
(43) Veröffentlichungstag der Anmeldung: 04.02.2009
(73) Patentinhaber: Beiersdorf AG, 20245 Hamburg (DE)
(72) Erfinder: Köhler, Manuela, 20144, Hamburg (DE); Neuhoff, Henrike, Hamburg, 22359 (DE); Wilken, Maren, 22848, Norderstedt (DE)

(56) Entgegenhaltungen:
- EP-A1- 1 925 286
- WO-A1-2006/118940
- DE-A1- 4 111 811
- DE-A1-102007 033 184
- JP-A- 4 295 416
- JP-A- 2003 081 768
- US-A- 6 106 816
- US-A1- 2005 220 829
- PROCTER AND GAMBLE: "Shimmery Spray Gel", INTERNET CITATION, 1. September 2006 (2006-09-01), Seiten 1-2, XP007915669, Gefunden im Internet: URL:http://www.gnpd.com/sinatra/gnpd/searc h_results/&item_id=587341 [gefunden am 2006-11-09]
- "ULTRA MILD CONDITIONING SHAMPOO WITH SUSPENDED MICA USING CARBOPOL ETD 2020 POLYMER", INTERNET CITATION, 28. November 2000 (2000-11-28), XP002305256, Gefunden im Internet: URL:http://www.personalcare.noveoninc.com/ formulations/EU0010.pdf [gefunden am 2004-11-11]
- NOVEON INC: "2-in-1 Conditioning Shampoo", INTERNET CITATION, 10. Februar 2006 (2006-02-10), Seiten 1-2, XP002521545, Gefunden im Internet: URL:http://web.archive.org/web/20061114133 318/http://www.personalcare.nov eon.com/formulas/SH-0020(EU).pdf [gefunden am 2009-03-27]

## Beschreibung

Die Erfindung betrifft Haarbehandlungsmittel in Form von Shampoo oder Haarspülung mit einem Gehalt an Diamanten, welche den Glanz des Haares verbessern.

Haarbehandlungsmittel sind hinlänglich bekannt.

Haarglanz ist für den Verbraucher eines der wichtigsten Produktanforderungen im Hair Care Bereich, denn Schönheit und Gesundheit wird mit glänzenden, attraktiv aussehenden Haaren verbunden. Daher sind kosmetische Produkte, die speziell zur Erhöhung des Haarglanzes beitragen für den Verbraucher von großem Interesse.

Der Glanz ist eine Eigenschaft, die dadurch zustande kommt, dass die Oberfläche eines Stoffes so glatt ist, dass Vertiefungen kleiner sind als die Wellenlänge des sichtbaren Lichtes. Der Glanz keratinischer Fasern hängt somit von der Oberflächenbeschaffenheit ab. Je rauer die Oberfläche (Cuticula), desto größer ist der Anteil an diffus reflektierten Licht und desto geringer ist der Glanz der Faser.

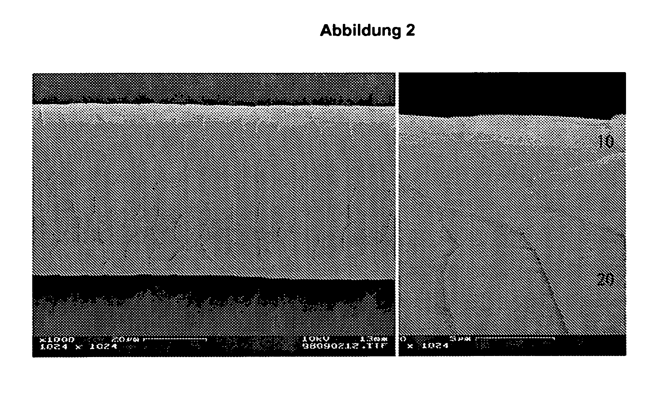

Das Haar lässt sich grob in zwei wesentliche Bestandteile unterteilen: das Haarinnere (lat. Cortex) und die dachziegelförmig um das Innere gelegte äußere Umhüllung des Haares (lat. Cuticula) (siehe Abbildung 2).

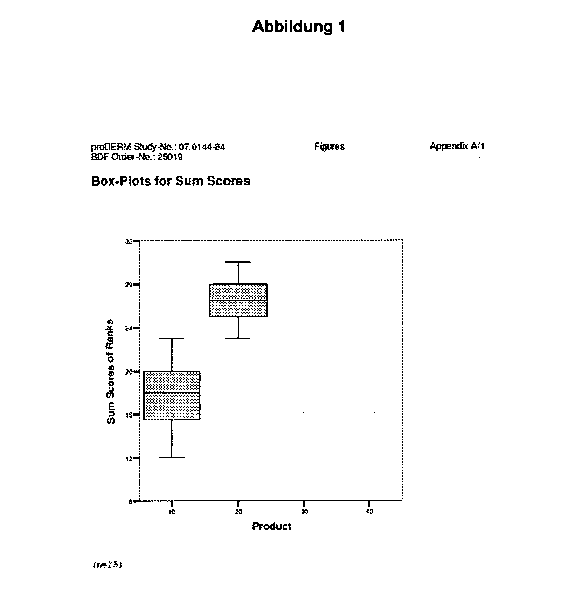

Die Cuticula besteht aus feinen, dachziegelartig geschichteten Schuppen, die verhornte Keratinozyten darstellen. Derart gleichmäßige Oberflächen findet man in der Regel an jungem Haar unmittelbar nach dem Austreten des Haares aus dem Haarschaft (siehe Abbildung 1).

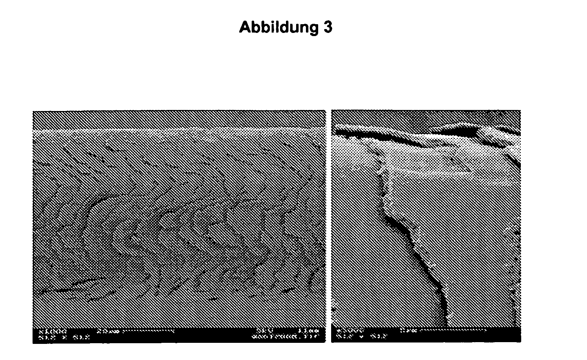

Die Cuticula bietet dem Cortex einerseits mechanischen und chemischen Schutz vor äußeren Einflüssen, andererseits Schutz gegen UV-Strahlen; sie verhindert das Austrocknen des Haares und die Entstehung von Spliss. Ferner ist die Oberfläche auch verantwortlich für das visuelle Erscheinungsbild, für den Glanz des Haares (siehe Abbildung 3).

Durch mechanische, chemische und umweltbedingte Einflüsse kommt es zur Veränderung und Schädigung des Haares, insbesondere der Cuticula. Die Cuticulaschuppen sind dann oft an den Enden abgebrochen und die einzelnen liegen nicht mehr eng an die Oberfläche an (siehe Abbildung). Solches Haar zeigt wenig Glanz.

Glanz keratinischer Fasern hängt folglich von der Glätte der Haaroberfläche ab. Zum Glätten der Haaroberfläche wurden bisher kosmetische Zubereitungen eingesetzt, in denen Öle, Wachse, Fette, Silikone oder pflegende Polymere eingesetzt werden, die eine hohe Affinität zum Haar haben und auf die Haaroberfläche aufziehen und diese dadurch glätten. Nachteil ist allerdings, dass diese Stoffe dem Haar oft ein fettiges Aussehen geben, es Beschweren und der damit erzielte Glanzeffekt höchstens bis zur nächsten Wäsche anhält. Pflegende Haarbehandlungsmittel werden beispielsweise in der WO 01/01934 A1, WO 00/61099, WO 2005107686 A1, US 20050158266 A1, EP 1569604 A1, US 20050112083 A1 beschrieben. In der DE 10058328 A1 wird z.B. ein Haarbehandlungsmittel mit einer quaternären Ammoniumverbindung und Fettalkoholen zur Haarglättung und Glanzerzielung beschrieben.

Darüber hinaus kann Haarglanz auch durch den Einsatz von Fruchtsäuren oder Panthenol erzielt werden. In der WO1998051264A1 wird beispielsweise ein Haarreinigungsmittel mit glanzverbessernden Eigenschaften beschrieben, welches zu 5 - 50 Gew% eines Tensids oder Tensidsgemisches, 2 bis 10 Gew.-% eines Gemisches von Fruchtsäuren und von 0,2 - 2 Gew% Panthenol oder eines seiner Derivate enthält. In der DE 19504914 C1 wird ein Haarwaschmittel, enthaltend mindestens ein anionisches, nichtionisches und/oder zwitterionisches (amphoteres) Tensid und ein Gemisch aus Milchsäure, Citronensäure; und Pyrrolidoncarbonsäure zur Verbesserung des Haarglanzes beschrieben.

WO2006 118940 beschreibt Haarbehandlungsmittel mit anorganischen Partikeln, die auch Diamant sein können.

Keine dieser Schriften offenbart aber erfindungsgemäße diamanthaltige Zubereitungen.

Diamant ist eine der kristallisierten Modifikationen des Kohlenstoffs. Er ist bei Oberflächenbedingungen gegenüber Graphit eigentlich thermodynamisch metastabil. Diamant hat eine Dichte von 3,52 t/m³ und eine Mohs-Härte von 10, er ist damit das härteste irdische Material. Diamanten können im Stahlmörser zu Pulver zerstampft werden.

Diamanten sind als hochwertige Schmucksteine bekannt. Eine höhere wirtschaftliche Bedeutung haben sie aber heute durch ihre industrielle Verwendung als Schneidstoffe und Schleifstoffe sowie als Zugabe in Polierpasten, wobei man sich ihre Härte und Verschleißfestigkeit zunutze macht.

Diamanten können auch synthetisch hergestellt werden. Dies gelang erstmals 1953/55. Die heutigen im thermodynamischen Stabilitätsbereich von Diamant operierenden Hochtemperatur-Hochdruck-Verfahren basieren auf der Umwandlung von Graphit in Diamant bei 1200 - 1400°C und 5 - 7 GPa Druck unter Mitwirkung geschmolzener Metalle als Lösemittel und Katalysatoren, meist Eisen, Cobalt, Chrom, Aluminium und Titan, früher auch Nickel. Dabei können durch Optimierung der Züchtungsbedingungen wie chemische Reinheit des eingesetzten Kohlenstoffs (z.B. ¹²C) und geeignete Zusammensetzungen der metallischen Flussmittel Stickstoff-arme Kristalle mit verbesserten optischen Eigenschaften hergestellt werden. 1990 wurde ein synthetischer Diamantkristall von 14,2 Karat Gewicht hergestellt. Industriell übliche Synthesen erfolgen mit dem Kohlenstoff-Isotop ¹²C. Seit 1991 können farblose, lupenreine Diamanten mit 3 Karat Gewicht aus 99% ¹³C hergestellt werden.

Mit dem Verfahren der CVD-Synthese (englisch chemical vapour deposition) lassen sich Diamanten auch bei Atmosphärendruck aus -1000°C heißen Gasen (Methan oder andere Kohlenwasserstoffe) in Gegenwart eines Wasserstoff-Überschusses abscheiden. Dabei entsteht ein nanometer- bis mikrometerdünner metastabiler Diamantfilm auf Metallsubstraten.

Nanokristalline Diamanten können durch intensive Bestrahlung von Graphit erhalten werden. Dabei entstehen zunächst zwiebelartige Schalenstrukturen aus Kohlenstoff-Atomen, die bei fortgesetzter Bestrahlung Bindungen zwischen den Schalen bilden und damit eine Selbstkompression verursachen, bei der Diamantkristallite gebildet werden. Mit einer laserbeheizten transparenten Diamantstempelzelle ist es heute möglich, die Druck-Temperatur-Bedingungen im Erdkern zu simulieren.

Eine Aufgabe dieser Erfindung bestand darin, ein Haarreinigungsmittel und Pflegeprodukt zu entwickeln, welches das Haar gründlich reinigt und pflegt und ihm zusätzlich einen intensiveren und lang anhaltenden Glanz verleiht als mit bisher erhältlichen kosmetischen Mitteln erreicht werden kann.

Es hat sich nun für den Fachmann unerwartet herausgestellt, dass durch den Einsatz von Diamantpartikeln in Hair Care Produkten keratinische Fasern nicht nur gründlich gereinigt und gepflegt werden, sondern der Glanz dieser Fasern intensiver und langanhaltend ist. Damit konnten für den Verbraucher spürbar bessere Produkte entwickelt und so den Nachteilen des Standes der Technik abgeholfen werden.

Diese erfindungsgemäßen Zubereitungen eignen sich hervorragend um Strukturen, wie z. B die Oberfläche keratinischer Fasern wieder glatt zu polieren und damit den Glanz dieser Fasern wieder lang anhaltend zu erhöhen.

Verwendet werden Diamanten mit einer mittleren Teilchengröße von 1 bis 20 µm, so genannte Diamant-Mikrokörnungen (erhältlich bei Firma Microdiamant). Als ganz besonders vorteilhaft hat sich eine mittlerer Teilchengröße von 4 - 6 µm gezeigt. Geeignete Einsatzkonzentrationen für den Diamanten liegen im Bereich von 0.0001-10 Gew.-%. Als bereits sehr wirkungsvoll haben sich Konzentrationen im Bereich von 0.001 von 1 Gew.-% gezeigt.

Wie aus Abbildung 1 ersichtlich, ergaben sich folgende Ergebnisse:
Glanzboxergebnisse erhoben bei ProDerm
Produkt 10: Shampooformel mit Diamantpuder
Produkt 20: Shampooformel ohne Diamantpuder
Produkt 10 gibt signifikant mehr Glanz als Produkt 20 (p<0.001*; α= 0.05) Wilcoxon signed ranks test of hair gloss.

Als Tenside können anionische, kationische, amphotere und nichtionische Tenside eingesetzt werden in Gesamtkonzentrationen von 2 bis 25 Gew.-%.

Besonders vorteilhaft zur Herstellung von Shampoos ist eine Kombination von Alkylethersulfaten (besonders Laurylethersulfat) und Cocamidopropylbetain, besonders bevorzugt Alkylethersulfate, Cocamidopropylbetain und Alkylpolyglykolestersulfosuccinate, ganz besonders bevorzugt Laurylethersulfat, Cocamidopropylbetain und Alkylpolyglykolestersulfosuccinateeinzusetzen. Besonders vorteilhaft ist Laurylethersulfat in Konzentrationen von 5 - 15 Gew.-% und Cocamidopropylbetain in Konzentration von 0.5 - 10 Gew.-% einzusetzen. Ganz besonders vorteilhaft ist es Laurylethersulfat in Konzentrationen von 6-12 Gew.-% und Cocamidopropylbetain in Konzentrationen von 1 - 5 Gew.-% einzusetzen.

Als ebenso sehr vorteilhaft hat sich die Kombination von Laurylethersulfat mit Cocamidopropylbetain und Decyl Glucosid erwiesen mit Konzentrationen von 2 - 12 Gew.-% Laurylethersulfat, 2 - 10 Gew.-% Cocamidopropylbetain und 2 - 5 Gew.-% Decyl Glucosid.

Ebenfalls vorteilhaft zu verwendende amphotere Tenside sind: Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat.

Im Fall von Haarspülungen und Haarkuren werden als Tenside kationische Tenside wie zum Beispiel Alkylamine, Alkylimidazole, Ethoxylierte Amine, Quaternäre Tenside, Esterquats eingesetzt.

Quaternäre Tenside erhalten mindestens ein N-Atom, das mit 4 Alkyl- oder Arylgruppen kovalent verbunden ist. Dies führt, unabhängig vom pH-Wert, zu einer positiven Ladung. Die erfindungsgemäß verwendeten kationischen Tenside können ferner bevorzugt gewählt werden aus der Gruppe der quaternären Ammoniumverbindungen, insbesondere Benzyltrialkylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylamidopropyltrimethylammoniumchlorid, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Monomere Ammonium-Verbindungen werden vielfach in Haarspülungen und Haarkuren z. B. in Konzentrationen von 0,5 - 5 Gew.-% eingesetzt. In leave on Produkte werden monomere oder polymere quartäre Ammonium-Verbindungen z.B. in Konzentrationen von 0,1 - 0,5 Gew.-% eingesetzt. Dazu gehören zum Beispiel neben anderen Ammonium-Verbindungen Cetrimonium Chloride wie es unter der Bezeichnung Dehyquart A von der Fa. Henkel angeboten wird oder Distearoylethyl Hydroxyethylmonium Methosulfate wie es unter der Bezeichnung Dehyquart F 75 von der Fa. Henkel angeboten wird.

Zur Pflege des Haares sind in dieser speziellen Zubereitung besonders vorteilhaft die Kombination der üblichen polymeren Haarkonditioniermittel (Polyquaternium-10, Guar Quats.). In Shampoos werden erfindungsgemäss zusätzlich vernetzte Polymere auf Acrylatbasis eingesetzt

Erfindungsgemäß vorteilhafte Polyacrylate sind Acrylat-Alkylacrylat-Copolymere, insbesondere solche, die aus der Gruppe der so genannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

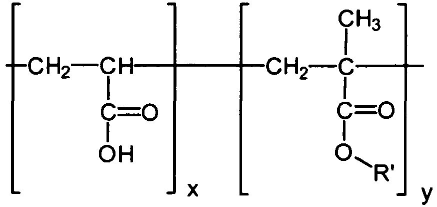

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit von der NOVEON Inc. als Carbopol ETD 2020 erhältlich.

Vorteilhaft sind Verbindungen, die die INCI-Bezeichnung "Acrylates/C 10-30 Alkyl Acrylate Crosspolymer" tragen.

Die Gesamtmenge an Polyacrylaten wird in den fertigen kosmetischen oder dermatologischen Zubereitungen vorteilhaft kleiner als 1,5 Gew.-%, bevorzugt zwischen 0,05 und 1,0 Gew.-%, bezogen auf das Gesamtgewicht der Zubereitungen, gewählt.

Es ist vorteilhaft im Sinn der vorliegenden Erfindung, wenn der Gehalt an einem oder mehreren Polyacrylaten in der kosmetischen oder dermatologischen Reinigungsemulsion aus dem Bereich von 0,1 bis 4 Gew.-%, ganz besonders vorteilhaft von 0,05 bis 1 Gew.-% gewählt wird, jeweils bezogen auf das Gesamtgewicht der Zubereitungen

Damit gelingt es besonders gut, die Diamantpartikel Partikel in der Schwebe zu halten, so dass diese nicht absinken. Bedingt geeignet sind nicht vernetzte Acrylatpolymere wie Carbopole oder Aqua SF-1, da diese insbesondere bei Abwesenheit von Silikonen zu einem unangenehmen, stumpfen Haargefühl führen. Ein Aspekt der vorliegenden Erfindung war es daher, vernetzte Acrylpolymere zu finden, so dass die Diamantpartikel stabilisiert und zugleich in Zusammenspiel mit den verwendeten Conditioning Polymeren eine angenehme Sensorik am Haar hinterlassen. Als besonders geeignet hat sich PAS2020 von Noveon herausgestellt.

Ein Aspekt der vorliegenden Erfindung war es, das vernetzte Acrylat einzuarbeiten. Dazu muss es erst auf pH 3-5 neutralisiert werden, um ausreichend gelöst zu werden. Die Conditioning Polymere können nicht sofort zugegeben werden, da sich ansonsten Gelklumpen bilden. Dies gilt insbesondere für PAS2020.

Als Verdicker werden bevorzugt Natriumchlorid, PEG-200 hydrogeniertes Glycerylpalmitat und Acrylat Copolymere verwendet. Besonders vorteilhaft sind Einsatzkonzentrationen von 0.05 - 5 Gew.-%.

Als Perlglanzsysteme können prinzipiell alle gängigen, bekannten Perlglanzrohstoffe verwendet werden. Besonders vorteilhaft ist die Verwendung von PEG-3 Distearat. Ganz besonders vorteilhaft ist es, wenn dieser Perlglanzrohstoff eine Teilchengröße aufweist in der Art, dass 90% der Volumenanteile eine Teilchengröße von kleiner 50µm aufweisen. Die Einsatzkonzentrationen des Perlglanzes liegen vorteilhafterweise bei 5 - 15 Gew.-%.

Anstelle des Perlglanzsystems kann auch ein Trübungsmittel eingesetzt werden. Besonders vorteilhaft ist der Einsatz von Mischungen, bestehend aus Glycol Distearat, Coco-Glucosid, Glyceryl Oleat, Glyceryl Stearate wie sie unter dem Handelsnamen Lamesoft TM Benz (Cognis) angeboten werden.

### Beispiele:

Folgende Handelsprodukte wurden eingesetzt:
Diamantpulver erhältlich bei Firma Microdiamant, Partikelgröße von 2,85 bis 18 µm. Mittlere Partikelgröße 5 µm.

### 1) Conditioner-Shampoo

| | 1 | 2 | 3 | 4 | 5 | 6 | 7 |
|---|---|---|---|---|---|---|---|
| Natrium Laurylethersulfat | 10 | 9 | 8 | 9 | 9 | 10 | 12 |
| Cocamidopropyl Betain | 1.5 | - | 3.5 | 3.5 | 4 | 4 | 3 |
| Di-Natrium PEG-5 Laurylcitrat Sulfosuccinat | - | - | 2 | 3 | 3 | - | - |
| Kokamid DEA | 3 | | | | | | |
| Decyl Glucoside | - | - | 3 | - | - | - | - |
| Natrium Cocoamphoacetat | - | 3 | - | - | - | - | - |
| Polyquaternium-10 | 0.1 | - | - | 0.3 | 0.3 | 0.2 | 0.1 |
| Polyquaternium-7 | - | - | 0.2 | 0.2 | - | - | 0.2 |
| Guar Hydroxypropyltrimoniumchlorid | 0.05 | 0.3 | 0.1 | - | 0.1 | - | - |
| Dimethicon | 1.3 | 1.5 | - | - | - | 2.0 | - |
| PEG-200 Hydrogenated Glyceryl Palmate | - | - | 0,2 | - | - | 1,0 | - |
| PEG-40 hydrogeniertes Rizinusöl | 0.2 | 0.3 | 0.4 | 0,5 | 0.6 | 0,5 | 0.5 |
| PEG-3 Distearat | 1.5 | - | 0.75 | 1.5 | 1.0 | - | 0.5 |
| Glycol Distearat | - | 4.0 | - | - | - | - | - |
| PEG-7 Glycerylcocoat | 0.5 | 1 | 2 | - | - | - | - |
| Natrium Salicylat | 0.2 | 0.3 | 0.4 | 0.4 | 0.4 | - | - |
| Natrium Benzoat | 0.5 | 0.4 | 0.3 | 0.2 | 0.4 | - | 0.5 |
| Parfüm | q.s. | q.s. | q.s. | q.s. | q.s. | q.s. | - |
| Benzophenon-4 | 0,2 | 0,25 | - | 0,3 | - | - | - |
| Oryzanol | - | - | - | 0,5 | - | - | 0,05 |
| Piroctone Olamine | - | - | - | - | - | 0.1 | 0.2 |
| Diamond powder | 0.005 | 0.01 | 0.001 | 0.002 | 0.01 | 0.05 | 0.1 |
| Niacinamid | - | 0.01 | - | - | - | - | - |
| Panthenol | - | - | 0.01 | - | - | - | 0.01 |
| Jojobaöl | - | - | - | - | 0,1 | - | - |
| Antedeschia Aethiopica Flower Extract | 0.001 | 0.01 | 0.1 | 0.2 | 0.5 | 0.005 | 0.02 |
| Mica | 0.05 | - | 0.01 | 0.03 | - | 0.05 | - |
| Natronlauge | q.s. | q.s. | q.s. | q.s. | q.s. | q.s. | - |
| Citronensäure | q.s. | q.s. | q.s. | q.s. | q.s. | q.s. | q.s. |
| Wasser | ad 100 | ad 100 | ad 100 | ad 100 | ad 100 | ad 100 | ad 100 |

### 2) Conditioner-Shampoo mit Perlglanz

| | 1 | 2 | 3 | 4 | 5 | 6 |
|---|---|---|---|---|---|---|
| Natrium Laurylethersulfat | 10 | 9 | 9 | 9 | 10 | 12 |
| Cocamidopropyl Betain | 1.5 | 3 | 4 | 3 | 4 | 3 |
| Dinatrium PEG-5 Laurylcitrat Sulfosuccinat | - | 3 | 3 | 2 | - | 4 |
| Kokamid DEA | 3 | - | - | - | - | - |
| PEG-200 Hydrogenated Glyceryl Palmate | - | - | 0.1 | - | 0,3 | 0,4 |
| Polyquaternium-10 | 0.1 | - | - | 0.3 | 0.3 | 0.2 |
| Polyquaternium-7 | - | - | 0.2 | 0.2 | - | - |
| Guar Hydroxypropyltrimoniumchlorid | 0.05 | 0.3 | 0.1 | - | 0.1 | - |
| Dimethicon | 1.3 | 1.5 | - | - | - | 2.0 |
| PEG-3 Distearat | 1.5 | 3 | 4 | 2 | 2,5 | 0,75 |
| Glycol Distearat | - | - | - | - | 0.5 | 0,5 |
| PEG-40 hydriertes Rizinusöl | 0.2 | 0.3 | 0.4 | 0.5 | 0.2 | 0,3 |
| Diamond powder | 0.005 | 0.01 | 0.001 | 0.1 | 0.01 | 0.05 |
| Natrium Salicylat | 0.2 | 0.2 | 0.4 | 0.4 | 0.4 | 0,2 |
| Natrium Benzoat | 0.4 | 0.4 | 0.4 | 0.4 | 0.4 | 0,4 |
| Natriumchlorid | 0,9 | 1,0 | 1,2 | 1,5 | - | 1,5 |
| Antedeschia Aethiopica Flower Extract | 0.001 | 0.01 | 0.1 | 0.2 | 0.5 | 0.005 |
| Mica | 0.05 | - | 0.01 | 0.03 | - | 0.05 |
| Natronlauge | q.s. | q.s. | q.s. | q.s. | q.s. | q.s. |
| Zitronensäure | q.s. | q.s. | q.s. | q.s. | q.s. | q.s. |
| Wasser | ad 100 | ad 100 | ad 100 | ad 100 | ad 100 | ad 100 |

### 3) Klares Conditioning-Shampoo

| | 1 | 2 | 3 | 4 | 5 | 6 |
|---|---|---|---|---|---|---|
| Natrium Laurylethersulfat | 10 | 9 | 3.5 | 3.5 | 0.5 | 8 |
| Natrium Myrethsulfat | - | - | 3.5 | 3.5 | 3.0 | 2 |
| Cocamidopropyl Betain | 1.5 | 4.5 | 3 | - | - | 3 |
| Natrium Cocoamphoacetat | - | - | - | 2.5 | - | 3 |
| Dinatrium PEG-5 Laurylcitrat Sulfosuccinat | - | - | - | - | 2.5 | 3 |
| Cocamide DEA | 3 | | | | | |
| Decylglucosid | - | - | - | 2 | 4.5 | - |
| PEG-200 Hydrogenated Glyceryl Palmate | 0.1 | - | - | 0.4 | 0.5 | - |
| Polyquaternium-10 | 0.1 | - | - | 0.3 | 0.3 | 0.2 |
| Polyquaternium-7 | - | - | 0.2 | 0.2 | - | - |
| Guar Hydroxypropyltrimoniumchlorid | 0.05 | 0.3 | 0.1 | - | 0.1 | - |
| Dimethicon | 1.3 | 1.5 | - | - | - | 2.0 |
| Diamond powder | 0.005 | 0.01 | 0.001 | 0.1 | 0.01 | 0.05 |
| Hydrolysiertes Seidenprotein | - | 0,1 | 0,2 | 0,3 | 0.3 | - |
| Antedeschia Aethiopica Flower Extract | 0.001 | 0.01 | 0.1 | 0.2 | 0.5 | 0.005 |
| Mica | 0.05 | - | 0.01 | 0.03 | - | 0.05 |
| PEG-40 hydriertes Rizinusöl | 0.1 | 0.2 | 0.3 | 0.1 | 0.2 | 0,4 |
| Natrium Salicylat | - | - | 0.4 | - | | 0,2 |
| Natrium Benzoat | 0.5 | 0.5 | 0.4 | 0.4 | 0.4 | 0,4 |
| Benzophenon-4 | - | - | 0.1 | 0,2 | 0,3 | 0,4 |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### 4) Mildes Baby Shampoo

| | 1 | 2 | 3 | 4 | 5 | 6 |
|---|---|---|---|---|---|---|
| Natrium Myristytethersulfat | 4 | 4 | 5 | 5 | 4 | 4 |
| Decylglucosid | 4 | 4 | 4 | 4 | 4 | 2 |
| Dinatrium PEG-5 Laurylcitrat Sulfosuccinat | 4 | 4 | 3 | 5 | 5 | 3 |
| PEG-80 Sorbitan Laurat | 2 | 1 | 1 | - | 0.5 | - |
| PEG-200 Hydrogenated Glyceryl Palmate | - | - | - | 0.2 | 0.3 | 0,4 |
| Polyquaternium-10 | 0.1 | - | - | 0.3 | 0.3 | 0.2 |
| Polyquaternium-7 | - | - | 0.2 | 0.2 | - | - |
| Guar Hydroxypropyltrimoniumchlorid | 0.05 | 0.3 | 0.1 | - | 0.1 | - |
| Dimethicon | 1.3 | 1.5 | - | - | - | 2.0 |
| Diamond powder | 0.005 | 0.01 | 0.001 | 0.1 | 0.01 | 0.05 |
| PEG-3 Distearat | - | - | 0,5 | 2 | - | - |
| Antedeschia Aethiopica Flower Extract | 0.001 | 0.01 | 0.1 | 0.2 | 0.5 | 0.005 |
| Mica | 0.05 | - | 0.01 | 0.03 | - | 0.05 |
| Glycol Distearat | - | - | - | - | 0.5 | 0,5 |
| PEG-40 hydriertes Rizinusöl | 0.2 | 0.3 | 0.4 | 0.2 | 0.3 | 0,4 |
| Natrium Salicylat | 0.2 | 0.2 | 0.2 | 0.4 | 0.4 | 0,4 |
| Natrium Benzoat | 0.4 | 0.4 | 0.4 | 0.4 | 0.4 | 0,4 |
| Natriumchlorid | 0.9 | 1.0 | 1.2 | 1,5 | 1,8 | - |
| Zitronensäure | q.s. | q.s. | q.s. | q.s. | q.s. | q.s. |
| Parfüm | q.s. | q.s. | q.s. | q.s. | q.s. | q.s. |
| Wasser | ad 100 | ad 100 | ad 100 | ad 100 | ad 100 | ad 100 |

### 5) Antischuppen Shampoo/Mildes Kopfhaut Shampoo (Formel 5)

| | 1 | 2 | 3 | 4 | 5 | 6 |
|---|---|---|---|---|---|---|
| Natrium Laurylethersulfat | 10 | 9 | 9 | 9 | 10 | - |
| Natrium Myristylethersulfat | - | - | - | - | - | 6 |
| Cocamidopropyl Betain | 1.5 | 4 | 3 | 3 | 4 | 5 |
| Dinatrium PEG-5 Laurylcitrat Sulfosuccinat | - | 3 | 3 | 3 | - | - |
| Natrium Cocoamphoacetat | - | - | - | - | - | 2.5 |
| Cocamide DEA | 3 | - | - | - | - | - |
| Decylglucosid | - | - | - | - | - | 2.5 |
| PEG-200 Hydrogenated Glyceryl Palmate | - | - | 0.1 | - | 0.4 | 3 |
| Polyquaternium-10 | 0.1 | - | - | 0.3 | 0.3 | 0.2 |
| Polyquaternium-7 | - | - | 0.2 | 0.2 | - | - |
| Guar Hydroxypropyltrimoniumchlorid | 0.05 | 0.3 | 0.1 | - | 0.1 | - |
| Dimethicon | 1.3 | 1.5 | - | - | - | 2.0 |
| Diamond powder | 0.005 | 0.01 | 0.001 | 0.1 | 0.01 | 0.05 |
| Climbazol | 0.5 | 0.5 | - | 0.5 | 1.0 | - |
| Antedeschia Aethiopica Flower Extract | 0.001 | 0.01 | 0.1 | 0.2 | 0.5 | 0.005 |
| Mica | 0.05 | - | 0.01 | 0.03 | - | 0.05 |
| Piroctone Olamin | - | 0.5 | 0.3 | - | 0.5 | - |
| Laureth-9 | - | - | - | - | 2 | 2 |
| Panthenol | - | - | - | - | - | 0,1 |
| Harnstoff | | | | 3 | 4 | 5 |
| PEG-3 Distearat | 1.5 | 3 | 0,75 | 0,5 | - | - |
| Glycol Distearat | - | - | - | - | 0.5 | - |
| PEG-40 hydriertes Rizinusöl | 0.2 | 0.3 | 0.4 | 0.5 | 0.2 | - |
| Natrium Salicylat | 0.4 | 0.4 | 0.4 | 0.4 | 0.4 | 0.2 |
| Natrium Benzoat | 0.4 | 0.4 | 0.4 | 0.4 | 0.4 | 0.4 |
| Natriumchlorid | 0.9 | 1.0 | 1.2 | - | - | - |
| Zitronensäure | q.s. | q.s. | q.s. | q.s. | q.s. | - |
| Milchsäure | - | - | - | - | - | q.s. |
| Parfüm | q.s. | q.s. | q.s. | q.s. | q.s. | q.s |
| Wasser | ad 100 | ad 100 | ad 100 | ad 100 | ad 100 | ad 100 |

### Haarkuren

| | 1 [%] | 2[%] | 3[%] | 4[%] | 5[%] | 6[%] | 7[%] |
|---|---|---|---|---|---|---|---|
| Hydroxypropylmethylcellulose | 0,5% | 0,5% | 0,5% | 0,3% | 0,4 % | 0,5% | 0,25% |
| Cetrimoniumbromid | 1,0 | - | 0,8 | - | 0,5 | 0,7 | 1,0 |
| Behentrimoniumchlorid | - | 0,7 | 0,3 | - | - | - | 0,1 |
| Distearoylethyl Hydroxyethylmonium Methosulfate | - | - | - | 1,2 | - | 0,7 | - |
| Palmitamidopropyltrimonium Chlorid | - | - | - | - | 0,5 | - | - |
| Glycerin | 3,5 | 8,5 | 3,0 | 2,5 | 2,0 | - | 8,5 |
| Cetearylalkohol | 2,5 | 2,0 | 3,5 | 2,5 | 2,0 | 3,5 | 4,8 |
| Glycerylstearat | 2,0 | 2,0 | 2,0 | 2,0 | 2,0 | 1,8 | 2,0 |
| Polyquaternium-10 | 0,1 | - | - | - | 0,1 | - | 0,1 |
| Guar Hydroxypropyl Trimonium Chlorid | - | 0,2 | - | 0,1 | - | - | - |
| Bis-Diglyceryl Polyacyladipate-2 | 2,0 | 2,0 | 2,0 | 2,0 | 2,0 | 2,0 | 2,0 |
| Diamond powder | 0,01 | 0,1 | 0,01 | 0,01 | 0,01 | 0,01 | 0,01 |
| Antedeschia Aethiopica Flower Extract | 0,1 | 0,1 | 0,1 | 0,01 | 0,1 | 0,1 | 0,1 |
| Panthenol | 0,1 | - | 0,1 | 0,1 | - | 0,15 | 0,15 |
| Oryzanol | 0,05 | 0,05 | 0,05 | - | 0,05 | 0,05 | 0,05 |
| Beads (Lactose, Cellulose, Cl 77007, Hydroxypropyl Methylcellulose) | 0,2 | - | 0,2 | 0,2 | 0,2 | 0,2 | 0,2 |
| Konservierungsmittel, Parfüm, pH-Einstellung und Lösungsvermittler | q.s. | q.s. | q.s. | q.s. | q.s. | q.s. | q.s. |
| Wasser | ad 100 | ad 100 | ad 100 | ad 100 | ad 100 | ad 100 | ad 100 |

### Haarspülungen:

| | 1[%] | 2[%] | 3[%] | 4 [%] | 5[%] | 6[%] | 7[%] |
|---|---|---|---|---|---|---|---|
| Cetrimoniumchlorid | 0,5 | 0,5 | 1,0 | - | 0,5 | 0,5 | 0,5 |
| Distearoylethyl Hydroxyethylmonium Methosulfate | - | - | - | 1,0 | - | 0,5 | 0,5 |
| Palmitamidopropyltrimonium Chlorid | - | - | - | - | 0,8 | - | - |
| Behentrimoniumchlorid | 0,3 | 0,2% | - | - | - | - | - |
| Cetearylalkohol | 2,8 | 2,5 | 3,3 | 3,0 | 2,6 | 2,8 | 2,8 |
| Glycerin | 8,5 | 3,0 | 8,5 | 2,8 | 2,5 | 2,0 | 8,5 |
| Hydroxyethylcellulose | 0,25 | 0,2 | 0,25 | 0,2 | 0,25 | 0,2 | 0,2 |
| Polyquaternium-10 | - | - | 0,1 | - | - | 0,1 | 0,1 |
| Guar Hydroxypropyl Trimonium Chlorid | - | 0,2 | - | - | - | - | - |
| Bis-Diglyceryl Polyacyladipate-2 | 2,0 | 2,0 | 2,0 | 2,0 | 2,0 | 2,0 | 2,0 |
| Diamond powder | 0,01 | 0,01 | 0,001 | 0,01 | 0,01 | 0,01 | 0,01 |
| Antedeschia Aethiopica Flower Extract | 0,1 | 0,01 | 0,1 | 0,01 | 0 | 0 | 0 |
| Panthenol | - | 0,15 | - | 0,15 | 0,1 | 0,15 | - |
| Oryzanol | 0,05 | 0,05 | 0,05 | 0,05 | - | 0,05 | 0,05 |
| Konservierungsmittel, Parfüm, pH-Einstellung und Lösungsvermittler | q.s. | q.s. | q.s. | q.s. | q.s. | q.s. | q.s. |
| Wasser | ad 100 | ad 100 | ad 100 | ad 100 | ad 100 | ad 100 | ad 100 |

### Leave on Kur

| | 1 [%] | 2 [%] | 3 [%] | 4 [%] | 5 [%] | 6 [%] | 7 [%] |
|---|---|---|---|---|---|---|---|
| Cetylalcohol | 1,5 | 1,8 | 2,0 | - | 1,0 | 1,7 | 2,0 |
| Cetrimoniumchlorid | 0,3 | 0,1 | 0,5 | 0,5 | - | 0,2 | - |
| Behentrimoniumchlorid | - | 0,2 | - | - | - | - | 0,2 |
| Distearoylethyl Hydroxyethylmonium Methosulfate | - | - | - | - | 0,5 | - | 0,2 |
| Palmitamidopropyltrimonium Chlorid | - | - | - | - | - | 0,2 | - |
| Benzophenone-4 | 0,05 | 0,03 | - | 0,1 | - | 0,1 | - |
| PVP/VA Copolymer (Luviskol VA 64 W) | 0,4 | - | - | - | 0,3 | - | - |
| Polyquaternium-37 | - | - | - | 1,0 | - | - | - |
| Polyquaternium-4 | - | - | - | 0,2 | - | 0,1 | - |
| Polyquaternium-10 | - | - | 0,5 | - | 0,3 | - | 0,2 |
| Panthenol | 0,1 | - | 0,2 | 0,1 | - | - | 0,2 |
| Hydroxyethylcellulose (Tylose H4000) | - | - | - | 0,3 | - | - | 0,2 |
| Acrylates/C10-30 Alkyl Acrylates Crosspolymer (Carbopol ETD 2020) | 0,5 | 0,3 | 0,2 | - | - | - | - |
| C12-13 Alkyl Lactate | 2,0 | 1,0 | 1,5 | 1,0 | 1,5 | 1,0 | 1,0 |
| Laureth-4 | - | - | - | 0,5 | - | - | - |
| Aluminium Starch Octenylsuccinate | - | - | - | 1,0 | - | - | - |
| Dicaprylyl Carbonate | - | - | - | 1,0 | - | - | - |
| Diamond powder | 0,01 | 0,01 | 0,01 | 0,01 | 0,01 | 0,01 | 0,01 |
| Antedeschia Aethiopica Flower Extract | 0,1 | 0,01 | 0,1 | 0,01 | 0 | 0 | |
| Panthenol | 0,1 | - | 0,1 | 0,15 | - | 0,15 | 0,1 |
| Oryzanol | 0,05 | 0,05 | - | 0,05 | 0,05 | 0,05 | - |
| Konservierungsmittel, Parfüm, pH-Einstellung und Lösungsvermittler | q.s. | q.s. | q.s. | q.s. | q.s | q.s | q.s |
| Wasser | ad 100 | ad 100 | ad 100 | ad 100 | ad 100 | ad 100 | ad 100 |

## Patentansprüche

1. Haarbehandlungszubereitung, vorliegend als Shampoo enthaltend Tensid und Diamant, **dadurch gekennzeichnet, dass** Diamant als Pulver vorliegt und die mittlere Teilchengröße der Diamantpartikel 1 bis 20 µm beträgt und vernetzte Polymere auf Acrylbasis enthalten sind.

2. Haarbehandlungszubereitung, vorliegend als Haarspülung enthaltend Tensid und Diamant, **dadurch gekennzeichnet, dass** Diamant als Pulver vorliegt und die mittlere Teilchengröße der Diamantpartikel 1 bis 20 µm beträgt und das Tensid ein kationisches Tensid ist, besonders bevorzugt gewählt aus der Gruppe Alkylamine, Alkylimidazole, ethoxylierte Amine, quaternäre Tenside, Esterquats.

3. Zubereitung nach Patentanspruch 1 oder 2, **dadurch gekennzeichnet, dass** die mittlere Teilchengröße der Diamantpartikel 4 bis 6 µm beträgt.

4. Zubereitung nach einem der vorangehenden Patentansprüche, **dadurch gekennzeichnet, dass** die Konzentration an Diamant 0,0001 bis 10 Gew.-%, bevorzugt 0,001 bis 1 Gew.-%, besonders bevorzugt 0,005 bis 0,1 Gew.-% beträgt.

5. Zubereitung nach einem der vorangehenden Patentansprüche, **dadurch gekennzeichnet, dass** Gehalt an Tensiden 2 bis 25 Gew.-% beträgt.

6. Zubereitung, vorliegend als Haarspülung nach einem der vorangehenden Patentansprüche **dadurch gekennzeichnet, dass** als Tensid eine Kombination von Alkylethersulfaten, besonders bevorzugt Laurylethersulfat, und Cocamidopropylbetain verwendet wird.

7. Zubereitung nach Patentanspruch 6, **dadurch gekennzeichnet, dass** zusätzlich Alkylpolyglykolestersulfosuccinate enthalten sind.

8. Zubereitung nach einem der vorangehenden Patentansprüche, **dadurch gekennzeichnet, dass** zusätzlich kationische Polymere enthalten sind.

9. Zubereitung nach einem der vorangehenden Patentansprüche, **dadurch gekennzeichnet, dass** zusätzlich Verdicker enthalten sind.

10. Zubereitung nach einem der vorangehenden Patentansprüche, **dadurch gekennzeichnet, dass** zusätzlich Perlglanzmittel oder Trübungsmittel enthalten sind.

11. Verwendung einer Zubereitung nach einem der vorangehenden Patentansprüche zur kosmetischen Behandlung keratinischer Fasern.

12. Verwendung einer Zubereitung nach einem der vorangehenden Patentansprüche zur Verbesserung des Glanzes von Haaren.

13. Verwendung einer Zubereitung nach einem der vorangehenden Patentansprüche zum Glätten des Haars.

## Claims

1. Hair treatment preparation, in the form of shampoo comprising surfactant and diamond, **characterized in that** diamond is present as powder and the average particle size of the diamond particles is 1 to 20 µm and acrylic-based crosslinked polymers are present.

2. Hair treatment preparation, in the form of hair rinse comprising surfactant and diamond, **characterized in that** diamond is present as powder and the average particle size of the diamond particles is 1 to 20 µm and the surfactant is a cationic surfactant, particularly preferably selected from the group alkylamines, alkylimidazoles, ethoxylated amines, quaternary surfactants, ester quats.

3. Preparation according to Patent Claim 1 or 2, **characterized in that** the average particle size of the diamond particles is 4 to 6 µm.

4. Preparation according to one of the preceding patent claims, **characterized in that** the concentration of diamond is 0.0001 to 10% by weight, preferably 0.001 to 1% by weight, particularly preferably 0.005 to 0.1% by weight.

5. Preparation according to one of the preceding patent claims, **characterized in that** the content of surfactants is 2 to 25% by weight.

6. Preparation, in the form of hair rinse according to one of the preceding patent claims, **characterized in that** a combination of alkyl ether sulphates, particularly preferably lauryl ether sulphate, and cocamidopropylbetaine is used as surfactant.

7. Preparation according to Patent Claim 6, **characterized in that** alkyl polyglycol ester sulfosuccinates are additionally present.

8. Preparation according to one of the preceding patent claims, **characterized in that** cationic polymers are additionally present.

9. Preparation according to one of the preceding patent claims, **characterized in that** thickeners are additionally present.

10. Preparation according to one of the preceding patent claims, **characterized in that** pearlizing agents or opacifiers are additionally present.

11. Use of a preparation according to one of the preceding patent claims for the cosmetic treatment of keratin fibres.

12. Use of a preparation according to one of the preceding patent claims for improving the shine of hair.

13. Use of a preparation according to one of the preceding patent claims for smoothing the hair.

## Revendications

1. Préparation de traitement capillaire, se trouvant sous forme de shampooing contenant un tensioactif et du diamant, **caractérisée en ce que** le diamant se trouve sous forme de poudre et la taille moyenne de particule des particules de diamant vaut de 1 à 20 µm et des polymères réticulés à base acrylique sont contenus.

2. Préparation de traitement capillaire, se trouvant sous forme d'après-shampooing contenant un tensioactif et du diamant, **caractérisée en ce que** le diamant se trouve sous forme de poudre et la taille moyenne de particule des particules de diamant vaut de 1 à 20 µm et le tensioactif est un tensioactif cationique, de façon particulièrement préférée choisi dans le groupe des alkylamines, alkylimidazoles, amines éthoxylées, tensioactifs quaternaires, esterquats.

3. Préparation selon la revendication 1 ou 2, **caractérisée en ce que** la taille moyenne de particule des particules de diamant vaut de 4 à 6 µm.

4. Préparation selon l'une quelconque des revendications précédentes, **caractérisée en ce que** la concentration de diamant vaut de 0,0001 à 10 % en poids, de préférence de 0,001 à 1 % en poids, de façon particulièrement préférée de 0,005 à 0,1 % en poids.

5. Préparation selon l'une quelconque des revendications précédentes, **caractérisée en ce que** la teneur en tensioactifs vaut de 2 à 25 % en poids.

6. Préparation, se trouvant sous forme d'après-shampooing, selon l'une quelconque des revendications précédentes, **caractérisée en ce qu'**on utilise comme tensioactif une association d'alkyléthersulfates, de façon particulièrement préférée de lauryléthersulfate, et de cocosamidopropylbétaïne.

7. Préparation selon la revendication 6, **caractérisée en ce que** des alkylpolyglycolester-sulfosuccinates sont en outre contenus.

8. Préparation selon l'une quelconque des revendications précédentes, **caractérisée en ce qu'**en outre des polymères cationiques sont contenus.

9. Préparation selon l'une quelconque des revendications précédentes, **caractérisée en ce qu'**en outre des épaississants sont contenus.

10. Préparation selon l'une quelconque des revendications précédentes, **caractérisée en ce qu'**en outre des agents nacrants ou des opacifiants sont contenus.

11. Utilisation d'une préparation selon l'une quelconque des revendications précédentes, pour le traitement cosmétique de fibres kératiniques.

12. Utilisation d'une préparation selon l'une quelconque des revendications précédentes, pour l'amélioration du brillant des cheveux.

13. Utilisation d'une préparation selon l'une quelconque des revendications précédentes, pour le lissage du cheveu.
